Europäisches Patentamt

European Patent Office

Office européen des brevets

(11) Veröffentlichungsnummer: **0 049 305**
B1

(12) **EUROPÄISCHE PATENTSCHRIFT**

(45) Veröffentlichungstag der Patentschrift:
21.05.86

(21) Anmeldenummer: 80106452.8

(22) Anmeldetag: 23.10.80

(51) Int. Cl.⁴: **A 61 H 13/00**, A 61 C 17/028

(54) Vorrichtung zum Erzeugen eines intermittierenden Sprays.

(30) Priorität: 30.09.80 DE 3036817

(43) Veröffentlichungstag der Anmeldung:
14.04.82 Patentblatt 82/15

(45) Bekanntmachung des Hinweises auf die Patenterteilung:
21.05.86 Patentblatt 86/21

(84) Benannte Vertragsstaaten:
AT BE CH FR GB IT LI LU NL SE

(56) Entgegenhaltungen:
DE - B - 1 053 741
DE - C - 481 034
DE - C - 552 375
DE - C - 831 868
FR - A - 558 763
FR - A - 687 741
NL - A - 7 905 465
US - A - 4 017 974

(73) Patentinhaber: ELMONT AG, Hauptstrasse 23,
CH-8280 Kreuzlingen (CH)

(72) Erfinder: Wild, Walter, Dr., Allmannsdorferstrasse 80,
D-7750 Konstanz (DE)

(74) Vertreter: Hiebsch, Gerhard F., Dipl.-Ing.,
Erzbergerstrasse 5A Postfach 464, D-7700 Singen 1 (DE)

LIBER, STOCKHOLM 1986

## Beschreibung

Die Erfindung betrifft eine Vorrichtung zum Erzeugen eines intermittierenden Sprays aus einem Flüssigkeits-Gas-Gemisch- insbesondere zur prophylaktischen und therapeutischen Anwendung im Dentalbereich - mit eine Flüssigkeit aufnehmendem Druckbehälter und in diesem oberhalb des Flüssigkeitsspiegels vorgesehenem Kopfraum, in dem sowohl ein die Flüssigkeit durchsetzendes Zuleitungsrohr für unter Druck stehendes Gas als auch ein in der Flüssigkeit endendes, nach unten offenes Tauchrohr münden, welches an einer zum Kopfraum hin offenen Mündung eines Ableitungsrohres für ein Gemisch aus Flüssigkeit und einem Teil des Gases endet.

Für die prophylaktische und therapeutische Behandlung von Zähnen finden sog. Mundduschen Anwendung, welche in der Regel eine kleine Wasserpumpe beinhalten, mit Hilfe derer das Wasser über einen Schlauch aus einer Düse in hartem, starkem Strahl herausgedrückt wird. Mit diesem Strahl wird zwar eine gewisse Reinigungswirkung erzielt, jedoch nur bis zum Zahnbett. Zudem verursacht der starke Strahl häufig Schmerzen, so daß die Benutzung der Munddusche unterlassen wird.

Anstelle einer Wasserpumpe wird in der Fraxis auch das Ausbringen der Flüssigkeit aus einem Behälter mittels Druckluft versucht. Dieses Verfahren hat jedoch die selben Nachteile wie oben genannt, zudem läßt die Druckluft im Laufe der Benutzung erheblich nach, so daß auch die Reinigungswirkung dieser Art von Mundduschen vermindert wird.

Zur Verminderung der Stärke des Wasserstrahls ist versucht worden, dem Wasserstrahl Luft beizumischen und so eine gewisse Art von Sprühnebel zu erzeugen. Dabei erfolgt die Zugabe von Luft an der Austrittsöffnung, d.h., an der Düse selbst. Dies bedeutet jedoch, daß bei der praktischen Handhabung ein Gefäß für Flüssigkeit und davon getrennt eine Vorrichtung zur Lufterzeugung vorhanden sein müssen, von welchen jeweils getrennte Ableitungen zur Düse führen. Diese Ausgestaltung ist nicht nur in der Handhabung äußerst umständlich, sondern auch sehr teuer. Zudem ist bei den bisher bekannten Vorrichtungen eine Einstellung des Luft/Flüssigkeits-Gemisches nicht möglich.

Eine Vorrichtung der eingangs erwähnten Art ist der DE-C-481 034 anhand einer heizbaren an einen Druckbehälter anschließbaren Handspritze für ärztliche, hygienische od. dgl. Zwecke zu entnehmen. In einem Glasbehälter münden zwei Leitungen in unterschiedlicher Höhe, zwischen denen ein Winkelrohr einer Sprühdüse verläuft. Über diesem Glasbehälter sitzt ein Medikantengefäß, dessen Inhalt dank eines Bodendurchbruchs in jenen Glasbehälter überführt und darin mit einem Gas oder einer Flüssigkeit gemischt werden kann.

Ausgehend von diesem Stand der Technik hat sich der Erfinder das Ziel gesetzt, eine derartige Vorrichtung so zu verbessern, daß sie mit intermittierendem Strahl betriebssicher arbeitet sowie kostengünstig herzustellen ist.

Zur Lösung dieser Aufgabe führt, daß das Ableitungsrohr für das Gemisch durch die Flüssigkeit hindurch ragt, am Tauchrohr anliegt und seine Mündung oberhalb des Flüssigkeitsspiegels der Öffnung des Tauchrohres benachbart ist, wobei dieser eine zu ihr koaxiale Stellschraube zugeordnet und in die Öffnung einführbar ausgebildet ist.

Nach einem weiteren Merkmal der Erfindung durchsetzt jene koaxiale Stellschraube eine den Druckraum des Druckbehälters nach oben hin schließende Querwand und ist mit einer diese übergreifenden Kappe lösbar verbunden sowie mit ihr drehbar. Zudem hat es sich als günstig erwiesen, in der Querwand eine Füllöffnung vorzusehen, welche der Mündung des Zuleitungsrohres gegenüberliegt sowie durch einen bewegbaren Ventildeckel des Zuleitungsrohres verschließbar ist.

Mittels dieser Vorrichtung wird auf sehr einfache Weise ein intermittierender Spray erzeugt. Gelangt nämlich ein Flüssigkeitstropfen in das vom Gas durchströmte Rohr, verengt sich kurzfristig dessen Querschnitt, was zu einer Erhöhung der Gasgeschwindigkeit innerhalb des Rohres führt. Infolgedessen läßt jedoch der auf die Flüsaigkeit wirkende Druck nach, es wird weniger Flüssigkeit gefördert, was wiederum zu einer Verringerung der Gasgeschwindigkeit im Rohr führt, wodurch wiederum der auf die Flüssigkeit wirkende Gasdruck erhöht wird. Mittels dieser einfachen Vorrichtung wird ein pulsierender Spray erzeugt, welcher sich besonders günstig auf die Behandlung des Zahnfleisches auswirkt.

Wichtig für die Wirkungsweise der Erfindung ist das Vorsehen eines Steuerorgans an dem Tauchrohr zum Regulieren der Flüssigkeitsmenge. Hierzu dient die beschriebene axiale Stellschraube, mittels deren Spitze der Querschnitt der Tauchrohrmündung verengt werden kann. Die Bewegung der Stellschraube wird dabei über einen am Deckel der Vorrichtung angeordneten Mitnehmer bewirkt.

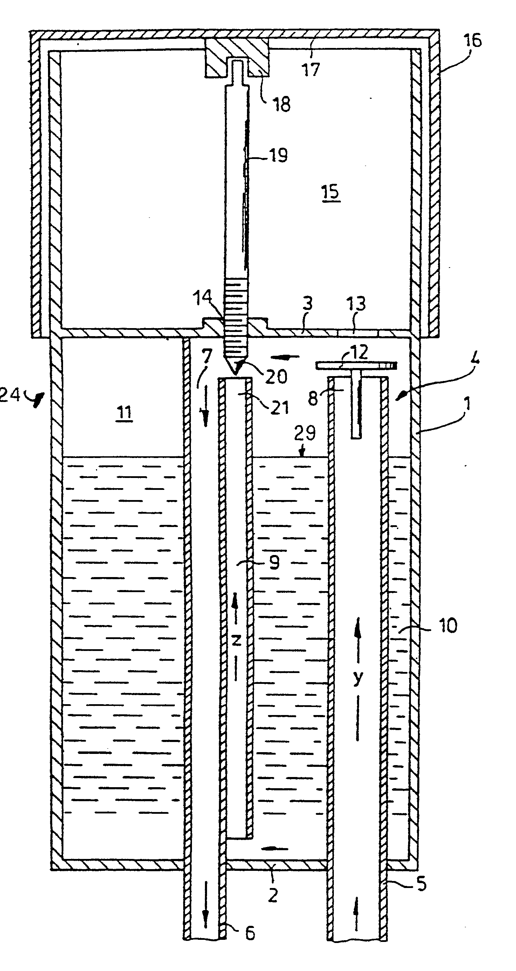

Weitere Vorteile, Merkmale und Einzelheiten der Erfindung ergeben sich aus der nachfolgenden Beschreibung eines bevorzugten Ausführungsbeispiels, das in der Zeichnung anhand eines Querschnitts dargestellt ist.

Gemäß der Figur umgibt ein zylindrischer Hohlkörper 1 mit Boden 2 und diesem gegenüberliegender Querwand 3 einen Druckraum 4, in dem ein Zuleitungsrohr 5 für Gas sowie ein Ableitungssrohr 6 für ein Gas/Flüssigkeits-Gemisch verlaufen; die rohrförmigen Leitungen 5, 6 durchsetzen den Boden 2, ihre oberen Mündungen 7, 8 sind nahe jener Querwand 3 vorgesehen.

In diesen Behälter 24 wird durch eine Wassereinlauföffnung 13 der Querwand 3 Flüssigkeit 10 eingefüllt, deren Spiegel 29 unterhalb jener Mündungen 7, 8 verläuft und auch unterhalb der Öffnung 21 eines Tauchrohres 9; letzteres erstreckt sich entlang des Ableitungsrohres 6 und seine Öffnung 21 ist jener des Ableitungsrohres 6 benachbart.

In Pfeilrichtung y in einem oberhalb des Spiegels 29 vorhandenen Kopfraum 11 eingeführtes Gas drückt auf die Flüssigkeit 10 und diese von unten her in das Tauchrohr 9, in dem sie in Pfeilrichtung z aufsteigt, sich an der Öffnung 21 mit Gas bzw. Luft mischt und dann durch das benachbarte Ableitungsrohr 6 als Flüssigkeit/Gas-Gemisch abgeführt wird.

Mit 12 ist ein Ventildeckel auf der Zuleitung 5 bezeichnet, der unterhalb jener Wassereinlauföffnung 13 der Querwand 3 lagert.

Die Wassereinlauföffnung 13 verbindet den Druckraum 4 mit einem weiteren Zylinderraum 15, welcher von einer Kappe 16 verschlossen wird. Von deren Deckelinnenfläche 17 ragt ein Mitnehmer 18 in den Zylinderraum 15, mit dem beim Drehen der Kappe 16 eine Axialschraube 19 verstellt wird; diese sitzt in einem Gewinde 14 der Querwand 3. Ihr unteres Konusende 20 ist der Öffnung 21 des Tauchrohres 9 zugeordnet und erlaubt je nach Lage eine stufenlose Veränderung des Öffnungsquerschnitts. Zum Füllen der Mischvorrichtung bzw. des Behälters 24 wird die Kappe 16 abgenommen und die Flüssigkeit 10 in den Zylinderraum 15 eingegossen. Diese tritt durch die Wassereinlauföffnung 13 in den Druckraum 4, wobei sie auf den Ventildeckel 12 prallt, welcher unter der Flüssigkeitslast das Zuleitungsrohr verschließt.

Bei Bedarf wird Gas unter Druck in das Zuleitungsrohr 5 eingeleitet, welches den Ventildeckel 12 anhebt und in den Druckraum 4 einströmt; der Ventildeckel 12 wird dabei gegen die Wassereinlauföffnung 13 gepreßt und verschließt diese. Dann beginnt die bereits angedeutete Förderung der Flüssigkeit 10 in Richtung z durch das Tauchrohr 9. An dessen Öffnung 21 wird die Flüssigkeit 10 zur Mündung 7 des Ableitungsrohres 6 umgelenkt, da durch diese Mündung 7 ein Teil des Gases abfließt. Auf diese Weise wird die Flüssigkeit 10 mit dem Gss vermischt sowie nach außen transportiert. Die Menge der der Mündung 7 zugeführten Flüssigkeit wird durch die Axialschraube 19 bzw. deren unteres Konusende 20 reguliert.

**Patentansprüche**

1. Vorrichtung zum Erzeugen eines intermittierenden Sprays aus einem Flüssigkeits-Gas-Gemisch, insbesondere zur prophylaktischen und therapeutischen Anwendung im Dentalbereich, mit eine Flüssigkeit aufnehmendem Druckbehälter (24) und in diesem oberhalb des Flüssigkeitsspiegels (29) vorgesehenem Kopfraum (11), in dem sowohl ein die Flüssigkeit durchsetzendes Zuleitungsrohr (5) für unter Druck stehendes Gas als auch ein in der Flüssigkeit endendes, nach unten offenes Tauchrohr (9) münden, welches an einer zum Kopfraum (11) hin offenen Mündung (7) eines Ableitungsrohres (6) für ein Gemisch aus Flüssigkeit und einem Teil des Gases endet,

dadurch gekennzeichnet,

daß das Ableitungsrohr (6) für das Gemisch durch die Flüssigkeit (10) hindurch ragt, am Tauchrohr (9) anliegt und seine Mündung (7) oberhalb des Flüssigkeitsspiegels (29) der Öffnung (21) des Tauchrohres (9) benachbart ist, wobei dieser eine zu ihr koaxiale Stellschraube (19) zugeordnet und in die Öffnung (21) einführbar ausgebildet ist.

2. Vorrichtung nach Anspruch 1, dadurch gekennzeichnet, daß die Stellschraube (19) eine den Druckraum (4) des Druckbehälters (24) nach oben hin schließende Querwand (3) durchsetzt und mit einer diese übergreifenden Kappe (16) lösbar verbunden sowie mit ihr drehbar ist.

3. Vorrichtung nach Anspruch 2, dadurch gekennzeichnet, daß in der Querwand (3) eine Füllöffnung (13) vorgesehen ist, welche der Mündung (8) des Zuleitungsrohres (5) gegenüberliegt sowie durch einen bewegbaren Ventildeckel (12) des Zuleitungsrohres (5) verschließbar ist.

**Claims**

1. A device for producing an intermittant spray consisting of a mixture of liquid and gas, especially for prophylactic and therapeutic application in the dental field, having a pressure reservoir (24) collecting a liquid (10) and a superior zone (11) provided in the pressure reservoir above the liquid level (29) in wich terminate both an inlet pipe (5) traversing the liquid and provided for gas under pressure and a plunger pipe (9) terminating in the liquid and being open towards the bottom, the plunger pipe terminating in an outlet being open towards the superior zone (11) of an outlet pipe (6) provided for a mixture of liquid and part of the gas,

characterized in that

the outlet pipe provided for the mixture traverses the liquid (10) and sits close to the plunger pipe (9), and that its outlet (7) is adjacent to the orifice (21) of the plunger pipe (9) above the liquid level (29), with a coaxial adjusting screw (19) being allocated to the opening (21) and designed for being introduced into the latter.

2. A device according to claim 1, characterized in that the adjusting screw (19) traverses a transverse wall (3) wich closes the pressure zone (4) of the pressure reservoir (24) towards the top and is both connected detachable with a cap (16) covering the wall and rotable with the cap.

3. A device according to claim 2, characterized in that a filling orifice (13) is provided in the transverse wall (3) which is situated opposite to the outlet (8) and is lockable by means of a movable valvet bonnet (12) of the inlet pipe (5).

## Revendications

1. Dispositif pour produire un jet pulverisé intermittent composé d'un mélange de liquides et de gaz destiné notamment à l'application prophylactique et thérapeutique dans le domaine dentaire comportant un réservoir sous pression (24) recueillant un liquide (10) et contenant une zone supérieure (11) prévue au-dessus de l'hauteur du liquide (29), dans laquelle débouchent et une conduite d'arrivée (5) traversant le liquide et destinée au gaz sous pression et un tube plongeur (9) ouvert au bas se terminant dans le liquide qui se termine à une bouche (7) ouverte vers la zone supérieure (11) d'une descente (6) pour un mélange composé de liquides et d'une partie du gaz.

caractérisé en ce que

la descente (6) destinée au mélange traverse et dépasse le liquide (10), colle au tube plongeur (9) et que sa bouche (7) voisine avec l'orifice (21) du tube plongeur (9) au-dessus de l'hauteur du liquide, à cette orifice étant adjoint une vis régulatrice (19) coaxiale fait pour être introduite dans celle-ci (21).

2. Dispositif selon la revendication 1 caractériséen ce que la vis régulatrice (19) traverse une paroi transversale (3) termant la zone sous pression (4) du réservoir sous pression (24) vers le haut et qu'elle est liée de manière détachable à un chapeau (16) s'étendant au-dessus de la paroi et qu'elle est orientable avec celui-ci.

3. Dispositif selon la revendication 2 caractérisé en ce que est prévue une orifice de charge (13) dans la paroi transversale (3) qui se trouve en face de la bouche (8) de la conduite d'arrivée (5) et qui est à fermer par un couvercle de soupape mobile (12) de la conduite d'arrivée (5).